# EUROPEAN PATENT SPECIFICATION

(11) **EP 0 470 231 B1**
(45) Date of publication and mention of the grant of the patent: **04.01.1995**
(21) Application number: 91904955.1
(22) Date of filing: 28.02.1991
(51) Int. Cl.: A61K 31/195

(54) **COMPOSITION AND USE OF THE COMPOSITION FOR THE MANUFACTURE OF A MEDICAMENT FOR THE TREATMENT OF INJURIES CAUSED BY THE RADIATION OF HEAT AND LIGHT**
ZUSAMMENSETZUNG UND VERWENDUNG DIESER ZUSAMMENSETZUNG ZUR HERSTELLUNG EINES ARZNEIMITTELS FÜR DIE BEHANDLUNG VON DURCH WÄRME- UND LICHTSTRAHLUNG VERURSACHTEN VERLETZUNGEN
COMPOSITION ET UTILISATION DE LA COMPOSITION POUR LA FABRICATION D'UN MEDICAMENT POUR LE TRAITEMENT DE LESIONS CAUSEES PAR LE RAYONNEMENT THERMIQUE ET CELUI DE LA LUMIERE THERMIQUE ET CELUI DE LA LUMIERE

(30) Priority: 28.02.1990 HU 117490
(43) Date of publication of application: 12.02.1992
(73) Proprietor: CHINOIN Gyògyszer és Vegyészeti Termékek Gyára RT., H-1045 Budapest IV (HU)
(72) Inventor: SZOCSIK, Katalin, H-1012 Budapest (HU); SZÜTS, Tamás, H-1027 Budapest (HU); VIRAG, Sándor, H-1136 Budapest (HU); Z.SZABO, Anna, H-1163 Budapest (HU); BENDEFFY, Erzsébet, H-1122 Budapest (HU); ORI, János, H-1019 Budapest (HU); SEBESTYEN, Gyula, H-1089 Budapest (HU); GERGELY, Vera, H-1055 Budapest (HU); KOVACS, Mária, H-1108 Budapest (HU); HIDASI, György, H-1142 Budapest (HU); JUHASZ, István, H-1225 Budapest (HU); DOBOZY, Attila, H-6721 Szeged (HU)
(74) Representative: Holmes, Michael John
(86) International application number: HU9100011
(87) International publication number: WO9112798

(56) References cited:
- GB-A- 1 503 674
- GB-A- 1 504 541
- GB-A- 2 050 164
- M. Negwer, "Organic-chemical drugs and their synonyms", Volume I, published 1987, by Akademie-Verlag (Berlin), see page 146, especially no.778

## Description

The present invention relates to the use of γ-L-glutamyl taurine (Litoralon^{R}) of the general formula I 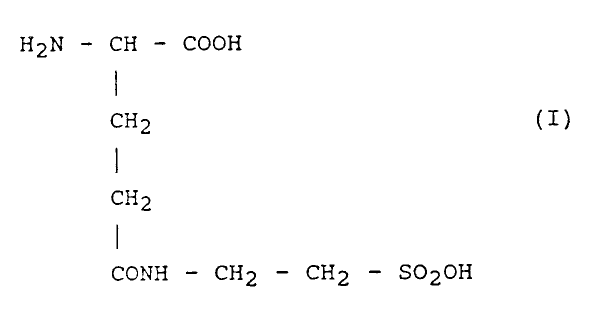
for the manufacture of a medicament or as a cosmetic for treating injuries and/or autoimmune diseases and skin lesion caused by heat or light radiation.

The harmful effect of heat and/or light radiation on the skin can often be observed. Furtheron under heat and/or light radiation the wave-length range between 3.10⁻³ cm to 3.10⁻⁷ cm is meant, which covers the whole infrared, visible and ultraviolet ray interval, the radiation may come from the sun or another optionally artificial source.

Dermatitis solaris is the most frequent skin disease, which appears in maculo papulose alterations all over the body in the form of erythema and urticaria.

Besides this "heat rash" often appears in the lack of direct sun light, mainly on infants and little babies. Furthermore, the sunlight exerts a direct injuring and/or provoking effect in case of systemic lupus erythematodes, pemphigus, recurring herpes simplex.

Presumably heat and/or light radiation, e.g. sunshine plays an important role in inducing of autoimmune processes, and this is proved by the seasonal manner of the appearance and inflammation of such processes.

For illustrating the harmful effect of sunshine the following statistical data are mentioned based on experiments carried out in the U.S.A. whereby the injuring effect of sunshine on the healthy population was assessed. During the examination carried out by the US National Health and Nutrition Examination Survey in 1971-74 20 637 persons were registered, 16191 of them were white and 4104 were black, in case of whom even a low dose of sunshine caused actinic transformations according to the following percental ratio:
white men 36.7 % v.s. black men 23.3 %
white women 34.1 % v.s. black women 18.6 %
Several efforts have been made so far to cure dermatitis solaris caused by the sunshine. In mild cases titanium dioxide, zinc oxide or talcum can be applied, in serious cases a systemic or local corticosteroidal treatment is often successful. In serious cases, when the inflammation of an autoimmune disease caused by the sunshine is expected, Chloroquin^{R}, Cyclosporin^{R}, Azationprint^{R} is used for prevention. Besides this, a low-dose PUVA and UV-B ray therapy is applied, which implies extremely high costs and long time, and which is just in a stage of experiment.

We found that skin injuries caused by the radiation of heat and/or light as well as the developing autoimmune diseases can be prevented and the pathological conditions already formed can be made better by the oral, topical or parenteral application of γ-L-glutamyl-taurine.

This is of great importance both for healthy people, and for those suffering from any disease of the immune system or from photodermatism caused e.g. by the permanent administration of some medicine. By medicines we mean e.g. azapropazone, bendrofluazide, chloropromazine, dichlofenac, phenoprofen, fenbufen, furosemide, hydrochloro thiazide, ibuprofen, malidix acid, pyroxicam and tetracyclines, each having a potential photosensitising effect [(British J. of Dermatology 121 551 (1989)]
The latter two groups form just a few percent of the population, however, their problem of being sensitive to the sunlight has been absolutely unsolved so far.

γ-L-glutamyl-taurine is a practically non-toxic endogenous prophylactic medicine and is suitable for the prevention or the treating of allergic reactions caused by the sunlight while having no influence on the positive effect of the sunlight on the health. Compounds of the invention do not prevent adsorption of the 280-315 nm ultra-violet range, but cease the injurious physiological processes started by the absorption of heat and light rays. The harmful effects of heat and light radiation on the skin and the immune system are not bound to any parts of the wave length UV range, but expectedly to many other intervals, too. Thus compounds of the invention are more favourable than the known UV absorbent light protecting compositions, both for healthy people and those suffering from an autoimmune disease or photophobia.

γ-L-glutamyl-taurine can be prepared by many processes, by isolation or synthesis (see BP 1.404.225, 1.503.674 and 1.504.541 and AP 379588).

In case of gamma and alpha radiation γ-L-glutamyl taurine showed a very little protective effect, however, it lowered the effective dose of known compounds decreasing the effect of ionizing radiation, e.g. aminoethyl-isotin sodium chloridehydrochloride (BP 2.050.164). This patent specification does not contain any reference to the prevention or treatment of pathological alterations caused by heat and/or light radiation by the application of γ-L-glutamyl taurine.

The minimal erythema dose (MED) in comparison to placebo was determined in accordance with the prescriptions of FOA (Federal Register, Vol 43 No. 166, August 25, 1978), and a considerable light protective factor was found at a 3x20 µg daily dose applied per os for treating lupus erithematodes, polymorph light exanthema, porphiria cutanea tarda and healthy people, by using light rays of 280-320nm and 320-400 nm range and the range of the sunlight.

### Clinical results:

28 men and 17 women (average age of 46.4 years) having a normal skin and according to previous observations inclined to the development of dermatitis solaris caused by sunlight were treated with γ-L-glutamyl taurine per os.

(For oral treatment Litoralon tablets, for topical treatment the hydrophilic ointment signed by b) were used, see later.)

The dosage was as follows: 2 days prior to the sunbathing Litoralon® tablets of 3x10 µg were taken for 2 weeks. In 68.9 % of the tested persons no actinic change, in 20 % slight change, and in 11.1 % actinic rashes similar to those of the previous years were observed.

Topic treatment was continued for 2 weeks in case of 8 men and 9 women, always before the sun-bathing. In healthy volunteers the areas characteristic of actinic alterations on the chest, upper arm and the face were treated with the hydrophilic ointment.

In 65 % of the cases the treatment had a very good effect, the rest of the cases could not be evaluated due to the short period of treatment or to the little number of cases, since the alleviation was moderate.

Furtheron the preparation of some γ-L-glutamyl taurine compositions suitable for oral or topical administration for prevention against heat and light is described.

Other medicines, medical cosmetics or cosmetical products, such as injections, syrups, suppositories, gels, plasters containing a compound of the formula I as well as medicines, medical cosmetics or cosmetical products containing a compound of the formula I and a known compound suitable for light protection compatible with γ-L-glutamyl taurine can also be prepared.

### 1. Litoralon tablets (active ingredient content of 0.01 mg)

| Content of 1000 tablets: | |
|---|---|
| Litoralon | 0,01 g |
| Lactose | 83.99 g |
| PVP | 3.00 g (polyvinylpyrrolidone) |
| Spiritus concentratis | 4.00 ml |
| Aqua destillata | 9.00 ml |
| Talcum | 2.00 g |
| Amylum solani | 10.00 g |
| Magnesium stearinicum | 1.00 g |

### Process of preparation:

I. Preparation of the granulating solution: polyvinylpyrrolidone (PVP) is dissolved in the mixture of 4 ml of alcohol and 6 ml of water.
II. The active ingredient is dissolved in some of the residual water, the mixture is added to the granulating solution and washed with the remaining water.
III. The auxiliary ingredients are sieved through a sieve of size 100.
IV. Lactose is homogenously wetted with the granulating solution containing the active ingredient as well.
V. Granulation through a sieve of size 16.
VI. Regranulation in a semi-wet state also through a sieve of size 16.
VII. Drying at room temperature, after which the moisture content should be below 1.5 per cent.
VIII. Addition of the outer phase:
A mixture of the following composition is added to the dry granulate:

| | |
|---|---|
| Amylum solani | 10.00 g |
| Talcum | 2.00 g |
| Magnesium stearinicum | 1.00 g |

IX. Preparation of the tablets: compressing is carried out with a flat instrument having a diameter of 7 mm and an edge.
Weight of 1 tablet: 0.10 g.

### 2. Compositions for topical application

The active ingredient content of the compositions is 0.1 mg per gramm.
a) Hydrophobic ointments containing Litoralon:
   I. 4.0 parts of cetylalcohol
      10.0 parts of wool fat
      85.9 parts of vaseline
      0.1 mg of active ingredient/g
   II. 10.0 parts of cetylalcohol
      10.0 parts of liquid paraffine
      79.0 parts of white vaseline
      0.1 mg of active ingredient/g.
b) Hydrophilic ointments containing Litoralon:
   I. 4.0 parts of Tween 80 (polyoxyethylene sorbitane mono-oleate)
      4.0 parts of liquid paraffine
      12.0 parts of cetylalcohol
      2.0 parts of preservative solution*
      57.9 parts of distilled water
      0.1 mg of active ingredient/g
   II. 47.5 parts of polyethylene glycol 400
      47.4 part of polyethylene glycol 4000
      5.0 parts of cetylalcohol
      0.1 mg of active ingredient/g ointment
      (This ointment can be washed off.)
   (Composition of the preservative solution: a mixture of parahydroxy-benzoic acid-methyl- and ethyl ester in a ratio of 40 to 60.)
   The components are homogenized and filled into tubes.
c) Creames containing Litoralon:
   Fatty: 12.0 parts of lanoline
   0.5 parts of cholesterol
   30.0 parts of sunflower or palm oil
   5.0 parts of white bees wax
   27.4 parts of vaseline
   23.0 parts of distilled water
   2.0 parts of preservative solution (of the same composition as described above)
   0.1 mg of active ingredient/g
   Semi-fatty:
   2.5 parts of lanata wax
   8.0 parts of lanoline
   10.0 parts of oleyl alcohol
   12.0 parts of stearine
   6.0 parts of white bees wax
   7.9 parts of propyleneglycol
   54.4 parts of distilled water
   0.1 parts of active ingredient/g
d) Powder:
   5.0 parts of zinc oxide
   10.0 parts of rice-starch
   84.9 parts of talcum
   0.1 g of active ingredient/g
   The components are admixed and the mixture is micronized.
e) Spray: 0.16 g of para-hydroxy-benzoic acid methyl ester
   8.0 g of distilled water
   32.0 g (40 ml) of anhydrous ethanol
   43.0 g (32.36 ml) of methylenechloride
   16.0 g of Entamol* (product of the West German firm Dehydag)
   0.1 mg of active ingredient/g
A vaseline-like substance, which protects the skin against drying.

## Claims

1. The use of γ-L-glutamyl-taurine of formula I 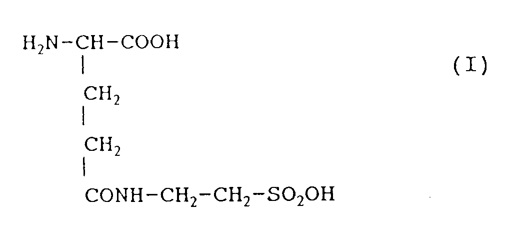 in the manufacture of a medicament to treat or prevent skin injuries and/or auto-immune diseases caused by heat or light radiation.

2. Use as claimed in claim 1 wherein said medicament is in an orally administrable form.

3. Use as claimed in claim 1 wherein said medicament is in a topically administrable form.

4. Use as claimed in claim 1 wherein said medicament is in a parenterally administrable form.

5. Use of a composition comprising γ-L-glutamyl-taurine of formula I 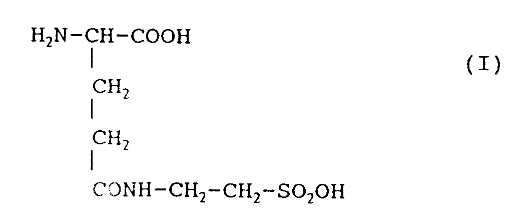 as a cosmetic for administration to skin conditions caused by heat or light radiation.

6. Use as claimed in claim 5, wherein said composition is administered orally, topically or parenterally.

## Patentansprüche

1. Verwendung von γ-L-Glutamyl-taurin der Formel 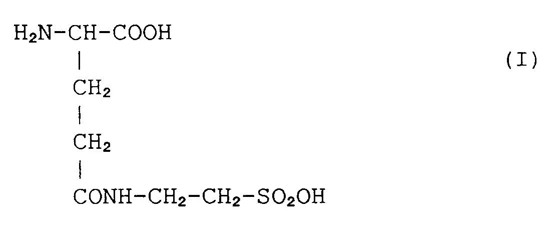 zur Herstellung eines Arzneimittels für die Behandlung oder Prophylaxe von Hautschädigungen und/oder Auto-immun-Krankheiten, die durch Hitze oder Lichtstrahlung verursacht worden sind.

2. Verwendung gemäß Anspruch 1, dadurch gekennzeichnet, daß das verwendete Arzneimittel in oral anwendbarer Form vorliegt.

3. Verwendung gemäß Anspruch 1, dadurch gekennzeichnet, daß das verwendete Arzneimittel in topisch anwendbarer Form vorliegt.

4. Verwendung gemäß Anspruch 1, dadurch gekennzeichnet, daß das verwendete Arzneimittel in parenteral anwendbarer Form vorliegt.

5. Verwendung einer Komposition, die γ-L-Glutamyl-taurin der Formel 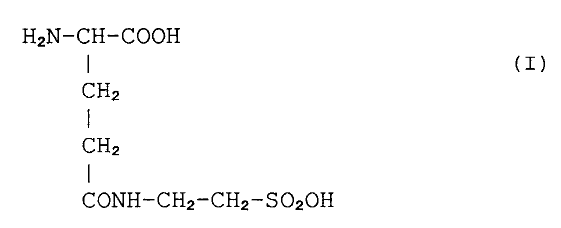 enthält, als Kosmetikum zur Behandlung von Hautaffektionen, die durch Hitze oder Lichtstrahlung verursacht worden sind.

6. Verwendung einer Komposition gemäß Anspruch 5, dadurch gekennzeichnet, daß die Komposition oral, topisch oder parenteral angewendet wird.

## Revendications

1. Application de γ-L-glutamyl-taurine de formule I 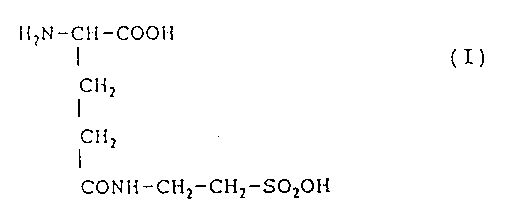 à la préparation d'un médicament pour traiter ou prévenir les atteintes dermatologiques et/ou les maladies auto-immunes provoquées par l'irradiation thermique ou lumineuse.

2. Application selon la revendication 1 dans laquelle ledit médicament est sous une forme administrable par voie orale.

3. Application selon la revendication 1 dans laquelle ledit médicament est sous une forme administrable localement.

4. Application selon la revendication 1 dans laquelle ledit médicament est sous une forme administrable par voie parentérale.

5. Application d'une composition comprenant la γ-L-glutamyl-taurine de formule I 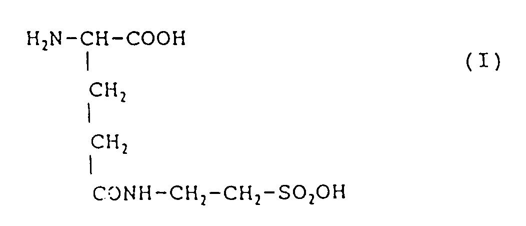 comme agent cosmétique aux fins d'administration dans les maladies dermatologiques provoquées par l'irradiation thermique ou lumineuse.

6. Application selon la revendication 5, dans laquelle ladite composition est administrée par voie orale, locale ou parentérale.
